# EUROPEAN PATENT SPECIFICATION

(11) **EP 0 957 774 B1**
(45) Date of publication and mention of the grant of the patent: **12.06.2002**
(21) Application number: 96905758.7
(22) Date of filing: 08.03.1996
(51) Int. Cl.: A61B 17/03, A61B 16/00

(54) **A DEVICE SUITABLE FOR USE IN CLOSING AN INCISION IN A BODY IN CONNECTION WITH AUTOPSY AND A METHOD FOR CLOSING SUCH INCISION**
VORRICHTUNG UND VERFAHREN GEEIGNET ZUM SCHLIESSEN EINES KÖRPEREINSCHNITTS WÄHREND EINER AUTOPSIE
DISPOSITIF ET PROCEDE ADAPTES POUR FERMER UNE INCISION DANS UN CORPS SOUMIS A UNE AUTOPSIE

(30) Priority: 20.03.1995 DK 28195
(43) Date of publication of application: 24.11.1999
(73) Proprietor: COLOPLAST A/S, 3050 Humlebaek (DK)
(72) Inventor: PEDERSEN, Jens, Kristian, DK-3100 Hornbaek (DK); OLSEN, Hans, Peter, DK-2970 Horsholm (DK)
(74) Representative: Bagger-Soerensen, Birgitte
(86) International application number: DK9600098
(87) International publication number: WO9629013

(56) References cited:
- US-A- 3 698 395
- US-A- 3 926 193
- US-A- 3 971 384
- US-A- 4 531 521
- US-A- 4 976 726

## Description

The present invention relates to a device suitable for use in closing an incision in a body in connection with autopsy, in particular a longitudinal incision along the centre line of the body, which device comprises at least one flexible sheet of material with a surface intended for facing the body and an opposite surface intended for facing away from the body, the body facing surface being provided with an adhesive, by means of which the device may be adhesively attached to the body in such a way that the device is present on both sides of the incision trace, and which device comprises two rows of mutually cooperating means for use in closing the incision, said rows of closing means being present on each side of the incision trace and allowing the incision to be made after adhesive attachment of the device, and a method for closing an incision in a body in connection with autopsy by use of such a device.

Incisions made in a body as a consequence of an accident or as part of a surgical procedure have traditionally been stitched together by means of needle and suture. This also applies to incisions made in connection with autopsy.

Up till now many different alternatives to this method have been proposed. F.inst. various devices of the staple type have been proposed.

Both staples and suture needles suffer from the drawback that they cause an additional damage of the tissue surrounding the incision and entail the risk of the needle or the staple penetrating the operation glove and damaging the operator's own tissue with resultant risk of infection, for instance by HIV. This is particularly a potential risk in connection with autopsy.

In addition various joining methods are known, using devices for the closing of an incision which are attached to the skin in the area around the incision by adhesion, see for instance the US Patents Nos. 3,698,395, 3,863,640, 3,926,193, 3,933,158, 3,971,384, 4,114,624, 4,531,521, 4,825,866, 4,966,605, 4,976,726, 5,026,389, 5,176,703, 5,234,462, and 5,263,970, of which only US 4,531,521 mentions that the device in question may be used in connection with autopsy.

Though it is mentioned in this US patent that the device may be used in connection with autopsy, the patent is, however, directed to a device for closing superficial incisions (skin separations), and it is specifically mentioned in the patent that subcutaneous suturing may be required in case of deep incisions.

It is an object of the present invention to provide a device and a method, by means of which a satisfactory closing of an incision in a body in connection with autopsy, and in particular of a longitudinal incision along the centre line of the body, may be obtained without the use of suture or staples.

In this connection it has turned out to be a particular problem to obtain a sufficiently tight closing of the incision in the neck and clavicular area of the body.

The problem is not only due to the fact that the body generally is curved in this area, but also that there are big differences from person to person as to the shape of the neck and clavicular area.

This problem is solved by the special design of the device according to the invention.

The invention thus relates to a device suited for use in closing an incision in a body in connection with autopsy, in particular a longitudinal incision along the centre line of the body, which device comprises at least one flexible sheet of material with a surface intended for facing the body and an opposite surface facing away from the body, the body facing surface being provided with an adhesive, by means of which the device may be adhesively attached to the body in such a way that the device is present on both sides of the incision trace, and which device comprises two rows of mutually cooperating means for use in closing the incision, said rows of closing means being present on each side of the incision trace and allowing the incision to be made after adhesive attachment of the device, which device is characterized in that said at least one sheet of material comprises a portion intended for being adhesively attached to the neck area of the body, which neck portion comprises an outwardly extending flap portion on each side of a line corresponding to the centre line of the body, and in that said at least one sheet of material at the end facing the neck portion comprises a portion, the side lines of which in a direction towards the neck portion substantially converge towards the line corresponding to the centre line of the body.

The outwardly directed flaps give in combination with the constricted part a configuration which enables the adaptation of the device to the contour of neck and clavicular areas of varying shape. At the same time the outwardly directed flaps give an increased surface which contributes to a better fixing of the device in the area in question, which is of vital importance, as the skin in this area is very movable, and as in particular this area is subjected to considerable forces during an autopsy.

In a preferred embodiment of the device according to the invention the flap portions extend obliquely outwardly and upwardly seen relative to a line corresponding to the centre line of the body and relative to the orientation of the body in upright position, respectively.

It is furthermore preferred that the outwardly extending flap portions have such a length that together with the intermediate part of the neck portion they span a distance of at least 25 cm, preferably at least 35 cm and particularly a distance of 40 - 55 cm. In the last-mentioned case the flaps may be able to reach each other in the back of the neck and even overlap somewhat, which gives a further safe securing of the device.

The ratio between the distance, which the outwardly extending flap portions and the intermediate part of the neck portion span, and the width of the portion with the substantially converging side lines, at the place, where the distance between them is smallest, will in most cases be about 1.5 or more.

In a particularly preferred embodiment the neck portion is designed as a separate unit. This makes a better utilization of the material possible, just as an additional possibility of adjustment is provided, the neck portion being thereby displaceable relative to the portion with the converging side lines.

In an embodiment which is particularly preferred if the neck portion is a separate unit, the neck portion is substantially trapezoidal, for instance designed as a trapezium where the distance between the parallel sides is of the magnitude 2 - 4 cm, and the corners at the base line of the trapezium are cut off, such that the neck portion in each side ends in a flap with a width of the magnitude 1 - 2 cm. The edges do not have to be completely straight. For instance one of the parallel sides may be slightly convex and the other slightly concave, just as only one of these sides may be slightly convex or slightly concave. Such a substantially trapezoidal, separately formed neck portion may depending on the shape of the neck and clavicular area be adhesively attached either with the short side upwards or with the short side downwards. Normally, it will, however, be designed with a view to being adhesively attached with the short side upwards.

In certain cases of autopsy there will not only be made an incision along the centre line of the body, but also a transverse incision in the neck area. In a particular embodiment of the device according to the invention the neck portion c is therefore provided with two rows of mutually cooperating closing means 7, 8, which extend into the outwardly directed flap portions 19, 20 for use in closing such an incision.

It is preferred to completely or partially design the device according to the invention with rounded corners to reduce the risk of detachment during handling of the body. This especially applies to the corners of the neck portion.

In a further preferred embodiment of the device according to the invention the portion with converging side lines is provided with notches or indentations in the edges running along these side lines in order to further improve the adjustment possibilities. These notches or indentations are preferably formed by removal of a part of the sheet of material, as there would otherwise be a risk of rejoining by cold flowing of the adhesive and of overlapping, when the release paper or foil is removed for adhesive attachment of the device. Likewise, the neck portion may be provided with a recess for receiving the curvature of a neck.

Opposite the neck portion said sheet of material comprises in general a substantially rectangular oblong portion, in which a part of the two rows of mutually cooperating means for use in closing the incision is present, said the means being positioned such that the two rows of means are present on either side of the intended incision trace and allow the incision to be made after adhesive attachment of the device.

It is preferred that the closing means cooperate in pairs, one means positioned on one side of the incision trace cooperating with a means positioned oppositely on the other side of the incision trace, and the two means of a pair, independently of the remaining pairs of cooperating means, may be connected operatively for closing the incision. This makes a stepwise closing of the incision possible.

It is further preferred that the closing means of a pair are provided with means for releasable locking of the closing means relative to each other during the closing operation. This makes it possible to carry out a gradual, controlled closing of the incision by alternating manipulation of the pairwise cooperating closing means, until the incision has been closed all over its length.

The ratio between the width of the rectangular portion and the width of the portion with the substantially converging side lines at the place, where the distance between them is smallest, will in most cases be about 1.2 or more, preferably about 1.5 or more and in particular 1.5 to 2.5.

In a preferred embodiment of the device according to the invention one closing means of a pair comprises a strap-like element, and the other closing means of a pair comprises a guiding device around or through which the strap-like element may be taken, such that a pull in the strap-like element will be able to produce an incision closing force substantially without producing any other impact on the area around the incision in the direction of the incision trace than what might be necessary for positioning the two closing means of a pair in line with each other.

The guiding device is preferably formed by an eye or a loop or a slot, through which the strap-like element may be passed. The guiding device does not have to be a closed member, but may also be a partially open member, provided it is able to carry out its guiding function both during the closing operation and during the influences, to which the device is subjected in the period, where the incision is to be kept closed by means of the device.

When in use the device according to the invention is attached adhesively to the body in such a way that the device is positioned on both sides of the incision trace. The device may, according to desire, be adhesively attached before the making of the incision or after the making thereof. It is, however, preferred to adhesively attach the device before making of the incision, such that the adhesive attachment is carried out on substantially dry skin. Especially in connection with autopsy is it an advantage to perform the adhesive attachment of the device before the making of the incision, as a better adhesion to skin is obtained and the least possible contact with bodily fluids is ensured, the risk of infection during the closing operation thereby being reduced.

To reduce the risk of damaging the strap-like element during the making of the incision the closing means with the strap-like element may be provided with means for keeping the strap-like element away from the incision trace during the making of the incision.

These means may be in form of a tap-taphole arrangement, whereby the strap-like element is kept backwardly bent, away from the incision trace. The hole is preferably formed in the strap-like element, whereas the tap is fastened to the sheet of material, preferably as a part of the closing means with the strap-like element.

Said arrangement is, however, only a single example of means for keeping the strap-like element away from the incision trace during the making of the incision, and a number of other embodiments of these means will. be possible. The strap-like element may for instance be kept away from the incision trace by means of a button-buttonhole arrangement, a resilient clamping device, a snap fastener, a strap, an adhesive, an adhesive tape, "Velcro" or any other arrangement which is able to keep the strap-like element away from the incision trace during the making of the incision without substantially impairing the functioning of the strap-like element and without entailing any unacceptable risk that the device according to the invention will detach itself from the skin during the release of the strap-like element.

In yet another preferred embodiment of the device according to the invention the closing means with the strap-like-element is provided with means for keeping the incision tool, such as a scalpel, away from the strap-like element during the making of the incision. These meams may f.inst. have the shape of one or preferably more protrusions on the closing means with the strap-like. element, which protrusions are preferably made integrally with the closing means, for instance by injection moulding.

During the closing of an incision by means of the closing means the area, in which the closing means are fastened to the flexible sheet of material, is subjected to considerable forces, which if the base of the closing means is not sufficiently large, may entail a risk of the subjacent skin delaminating. Therefore, in a preferred embodiment the width of the closing means base is at least 15 mm, preferably at least 20 mm and in particular at least 25 mm, and the length of the closing means base is at least 15 mm, preferably at least 20 mm and in particular at least 25 mm. Furthermore, it is preferred that the corners of the closing means base are rounded.

In another preferred embodiment the mutually cooperating closing means are arranged with a mutual spacing of at least 15 mm, preferably at least 20 mm, in a direction transversely to the incision trace. Hereby, it becomes possible not just to bring the tissue together in the position, in which it was prior to the making of the incision, but to perform an additional clamping together, whereby the incision edges with the adhesively attached flexible sheet of material and the subjacent skin are bent down into the incision area, and the upper surface of the edge areas of the sheet of material on each side of the incision trace abut each other and thereby contribute to an improved sealing. In this connection it is a particular advantage if the edge area of the base of the closing means facing the incision trace is flexible.

In a further preferred embodiment one of the closing means comprises a strap-like element, which is provided with at least two ribs or cams, one of which corresponding to a fully closed position and another corresponding to an intermediate position, and the other one of the closing means comprises a catch buckle. The strap-like element and the catch buckle are advantageously fastened at least 2 mm away from the edge of the base of the closing means facing the incision trace, in particular if the edge area of the base of the closing means facing the incision trace is flexible. With this embodiment it has turned out to be possible to manufacture closing means having a total thickness in closed condition of below 3 mm.

In another embodiment the mutually co-operating closing means comprise guiding devices which are positioned on either side of the incision trace, and separate, strap-like elements, by means of which the mutually cooperating closing means pairwise may be brought into operative connection with each other for closing of the incision, said strap-like elements being provided with a stopping member adapted to prevent the strap-like element from passing transversely through the guiding device on one side of the incision trace and means for bringing it into locking engagement with the guiding device on the other side of the incision trace in at least one position.

The guiding device may f.inst. be a catch buckle, and the strap-like element be provided with one or more ribs or cams which may be brought into engagement with the catch of the catch buckle.

In another preferred embodiment the means are designed in such a way that when the two closing means of a pair have been brought into operative connection, it is possible by a one hand pull in one of the means of the pair to apply to both of the means of the pair in a direction towards each other simultaneously acting incision closing forces substantially without any other impact on the area around the incision in the direction of the incision trace than what might be necessary for bringing the two means of a pair in line with each other.

Owing to the fact that when the two closing means of a pair have been brought into operative connection with each other it is possible by a one hand pull in one of the means of the pair to apply to both of the means of the pair in a direction towards each other simultaneously acting incision closing forces, the operator becomes able to have one hand free to bear against the body itself, while the incision surfaces are moved towards each other through a pull by the other hand. As the closing means of a pair moreover are subjected to in a direction towards each other simultaneously acting incision closing forces substantially without any other impact on the area around the incision in the direction of the incision trace being produced than what might be necessary for positioning the two means of a pair in line with each other, it becomes possible to obtain a closing of the incision with a minimum influence on the surrounding tissue, which may be of considerable importance in connection with the closing of an incision in connection with autopsy.

The cooperating closing means of a pair may as mentioned be provided with means for releasable locking of the closing means relative to each other during the closing operation. These means may for instance be of the type a catch buckle, a belt buckle, a hook buckle, a button-buttonhole, a tap-taphole, a snap fastener or "Velcro".

In an embodiment of the device according to the invention the means for releasable locking of the closing means of a pair are provided on the closing means with the strap-like element. When closing an incision by use of a device of this design, the strap-like element is passed around or through the guiding device on the other means of the pair and back towards the means with the strap-like element, where it is brought into operative connection with the means for releasable locking of the closing means of a pair being located on the closing means with the strap-like element. These means may optionally be the same as the means used for keeping the strap-like element away from the incision trace during the making of the incision. The strap-like element may for instance be provided with a row of holes, by means of which the strap-like element may be fastened to a tap of suitable design. This tap may for instance be directed backwardly relative to the incision trace, such that a continued pull in the strap-like element will release a hole from the tap and optionally bring one of the succeeding holes in engagement with the tap for locking in this position. Also other means for releasable locking of the closing means of a pair, such as means of the above-mentioned types, may be utilized.

In an alternative embodiment the strap-like element is not fastened to the closing means comprising it, but is instead fastened to the closing means with the guiding device after having been passed around or through it, the means for releasable locking of the closing means in a pair partially being provided on the strap-like element and partially on the closing means with the guiding device. The guiding device may form part of a buckle construction.

To reduce the risk of the device detaching from the skin it is preferred that the locking of the closing means in a pair relative to each other is releasable substantially without exerting any pulling forces in a direction perpendicular to the plane of the sheet of material, and in particular that the locking is releasable by a continued pull in one of the means of the pair.

Examples of locking mechanisms, the locking of which is releasable by a continued pull in one of the closing means of the pair, are mechanisms of catch buckle, belt buckle, hook buckle and certain kinds of tap-taphole types.

In a presently preferred embodiment of the device according to the invention, one closing means of the pair of cooperating means comprises a strap-like element which is hinged in a fixed part and which is on one side provided with a row of transverse ribs, and a locking mechanism in form of a tap-taphole arrangement, by means of which the strap-like element may be kept in a backwardly bent position, until the incision has been made, and the fixed part is provided with a pair of protrusions extending in direction towards the incision trace for protection of the strap-like element during the making of the incision, and the other means of the pair is a catch buckle.

In an embodiment of the device according to the invention the sheet of material is continuous in the area, where the incision is to be made. The device may, however, also be divided in two parts intended for being adhesively attached on each side of the incision trace, one part being provided with one row of the mutually cooperating closing means and the other part with the other row of the mutually cooperating means, the two parts being adhesively attached in such a way that the means will be positioned pairwise opposite one another on each side of the incision trace. Furthermore, as previously mentioned, the neck portion may be formed as a separate unit.

It is preferred that the sheet of material is continuous in the area, where the incision is to be made, the most exact positioning of pairwise cooperating closing means relative to each other thereby being ensured. The incision may then be made through the sheet of material.

The sheet of material will typically be made from one of those materials which are commonly used for plasters. The sheet of material may for instance be made from a foil of polyurethane, EVA (ethylene vinyl acetate) with a high vinyl acetate content, chlorinated polyethylene or plasticized PVC, polyurethane being the preferred material for the time being. A particular requirement regarding the sheet of material is that it should be of adequate flexibility and elasticity to be able to abut the skin in the incision area to a sufficient degree. For use in closing an incision in connection with autopsy it should also have sufficient water tightness to be able to exert the desired sealing function.

A presently preferred sheet of material is a polyurethane foil with a weight of 10 to 100 g/m² and preferably with a weight of 20 to 50 g/m². For instance, a polyurethane foil with a weight of about 35 g/m² and a breaking strength of more than 7.5 N/25 mm and an elongation at break of more than 300% has turned out to be suitable.

The adhesive used is preferably an elastic adhesive which is able to absorb a certain amount of moisture like for instance a hydrocolloid-containing adhesive. A preferred adhesive of this type is the adhesive described in DK patent publication No. 147,035 B (corresponding to GB patent publication No. 2,089,351 B), which adhesive comprises one or more water-swellable hydrocolloids dispersed in a continuous phase containing a physically cross-linked elastomer in the form of one or more styrene-olefin-styrene block copolymers, a polar plasticizer for the elastomer, which is compatible with at least its styrene blocks and which lowers the glass transition temperature of the styrene blocks in the elastomer, a hydrocarbon tackifier resin in the form of a polymer or copolymer of cyclopentadiene, dicyclopentadiene, α-pinene and/or β-pinene, an antioxidant and possibly an oil extender consisting of one or more mineral oils. A further example of a hydrocolloid-containing adhesive which would be suitable is the adhesive described in WO 96/00094. Also other corresponding adhesives may be used.

Until the device is used, the adhesive will usually be covered by a sheet of release paper or foil on the body facing surface. These materials are well known within the present field and will not be described in detail here.

As will be understood, among others depending on the distance between the pairs of cooperating closing means and the contour of the body, larger or smaller areas may occur, in which areas the cutting surfaces of the incision do not abut completely after the closing operation. This may for instance be the case when closing an incision along the centre line of the body in connection with autopsy. It may therefore be desirable to make an additional sealing by adhesively attaching over the incision line a flexible further sheet of material of such dimensions that it may be brought to cover the incision area and preferably also the closing means. In said particular case it is preferred that the additional sheet of material is skin-coloured and comparatively nontransparent. This further sheet of material is on one side provided with a layer of adhesive, said layer of adhesive being covered by a sheet of release paper or foil. By adhesively attaching such a further sheet of material it is possible to acquire a completely liquidproof connection.

In a particular aspect the invention thus relates to a set comprising a device according to the invention, as well as a flexible, preferably skin-coloured and preferably nontransparent further sheet of material of such dimensions that after closing of an incision by means of said device it may cover the incision area and preferably also the closing means, a layer of adhesive having been applied to one surface of said further sheet of material, said adhesive layer being covered by a sheet of release paper or foil.

In a variant thereof the set comprises two further sheets of material, the first one of which having such dimensions that after closing of an incision by means of said device it may cover the incision area, and the other one having such dimensions that after closing of an incision by means of said device and after the first further sheet of material having been adhered in the incision area, the closing means can be covered.

In a preferred embodiment the set comprises a further sheet of material of such dimensions that after closing of an incision by means of the device it is capable of substantially covering the incision area and the closing means, which further sheet of material has rounded corners at the end intended to face the neck and at this end is provided with notches or indentations in the side edge. To avoid an unintentional sticking together of the notches or the indentations by cold flowing of the adhesive or by overlapping, when the release paper or foil is removed for adhesive attachment of the device, the notches or the indentations are preferably formed under removal of material from the sheet of material.

As adhesive for the further sheet(s) of material a hydrocolloid-containing adhesive as described above is preferably used. An adhesive of this type has the advantage of being able to absorb moisture and bodily fluids through a period of time without losing its adhesiveness. This ability to absorb moisture is of particular importance in connection with autopsy, where the body often has a lower temperature than the surroundings, which may give rise to condensation on the body of moisture from the air.

A particularly good absorption of moisture may be obtained when the further sheet of material or at least one of the further sheets of-material immediately above the incision area has been provided with an adhesive layer which is thicker, preferably at least twice as thick and in particular 3 - 5 times as thick as the layer of adhesive on the device itself.

Bodies, on which an autopsy has been performed, and which have been closed by means of a set as described above, in which both sheets of material have been provided with a hydrocolloid-containing adhesive, have thus turned out to be manageable still after one week, which must be considered a sufficiently long time.

For the manufacture of the further sheet(s) of material the same types of material may be used as those used for the manufacture of the first sheet of material. However, the further sheet of material or at least one of the further sheets of material will often be coloured as mentioned above, such that the connection itself becomes invisible after the adhesive attachment of the further sheet of material.

The closing means may typically be made by injection moulding, powder pressing, punching or the like and may for instance be made from a thermoplastic material like nylon, polypropylene, polyethylene or a similar material with adequate mechanical properties for tolerating the mechanical forces to which the closing means are exposed. In a presently preferred embodiment the closing means are made from polyethylene.

The closing means will typically be fastened to the sheet of material by welding, gluing or adhering, for instance by means of an acrylate adhesive, but any other method of joining, which gives a sufficient fastening of the closing means to the sheet of material without weakening the function of any of these parts to an unacceptable degree, will be usable in principle. Thus, in principle, there is nothing impeding the making of the closing means integrally with the sheet of material.

After the manufacture the device may if desired be sterilized and packed in a conventional manner, the sterilization being for instance made by radiation or by treatment with ethylene oxide.

Finally, the invention relates to a method for closing an incision in a body in connection with autopsy, in particular a longitudinal incision along the centre line of the body, which method is characterized in that before making the incision a device according to the invention is adhesively attached to the body in such a way that the device is positioned on either side of the intended incision trace, with the two rows of mutually cooperating closing means being positioned on either side of the incision trace and the neck portion being adhesively attached in the neck area of the body with the outwardly directed flaps placed around the neck, possibly in such a way that they overlap, that after the making of the incision the mutually cooperating closing means are brought into operative connection with each other for closing the incision, the pairwise cooperating closing means being, if desired, operated alternately for a controlled gradual closing of the incision under releasable locking in at least one intermediate position, and the closing operation being, if desired, continued beyond the starting position such that the incision edges with the adhesively attached sheet of material and the subjacent skin bend downwardly into the incision area and such that the edge areas of the sheet of material on either side of the incision trace abut, and in that, if desired, after possible wiping of the upper side of the device, one or more further sheets of material as stated above is adhesively attached for covering the incision area and preferably also the closing means.

The invention will be described in detail in the following with reference to the drawing, in which
Fig. 1 schematically shows a device according to the invention,
Fig. 2 is a schematic sectional view along the line II-II in Fig. 1,
Fig. 3 shows a pair of the mutually cooperating means for closing of the device in Fig. 1,
Fig. 4 is a sectional view along the line IV-IV in one of the closing means shown in Fig. 3,
Fig. 5 shows the closing means shown in Fig. 3 in closed condition,
Fig. 6 is another embodiment of a pair of mutually cooperating closing means,
Fig. 7 shows the closing means shown in Fig. 6 in a partially closed condition,
Fig. 8 schematically shows the device according to Fig. 1 with a further sheet of material adhesively attached, said sheet covering a performed incision and the closing means used for closing the incision, and
Fig. 9 - 12 schematically shows a set according to the invention comprising another embodiment of a device according to the invention, with the neck portion designed as a separate unit, and with two further sheets of material, one of which being intended for covering the incision area after the closing of the incision, and the other being intended for covering both the first further sheet of material and the closing means.

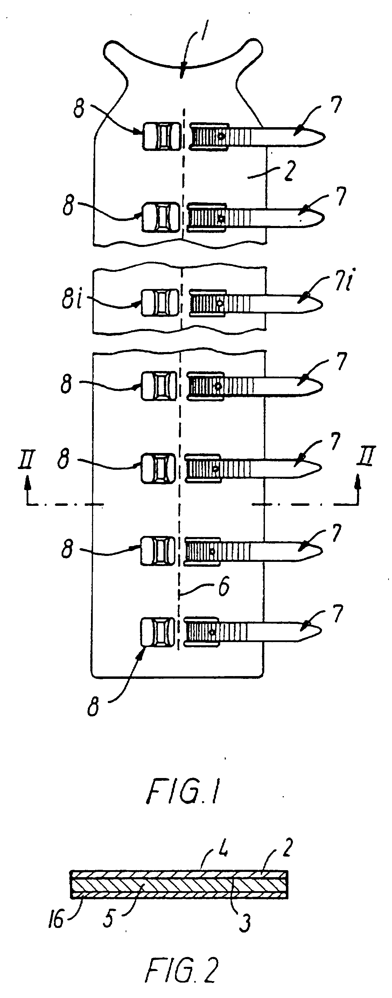

Fig. 1 shows schematically a device 1 according to the invention which is particularly suited for use in closing an incision in a body in connection with autopsy. For said purpose the width of the device will typically be 12 to 20 cm and the length 60 to 80 cm, but the device might also have been made with a length up to 90 cm or up to 100 cm. If the device is too long for fitting a given body, the surplus length may simply be cut off. As schematically illustrated in Fig. 2 which is a sectional view along the line II-II in Fig. 1, the device comprises a flexible sheet of material 2 with a surface 3 which is intended to face the body, and an opposite surface 4 which is intended to turn away from the body. An adhesive 5 has been applied to the body facing surface, said adhesive being covered by a sheet of release paper or foil 16, for instance in form of waxed or siliconized paper or foil. After removal of the release paper or foil the device 1 is adhesively attached to the body in such a way that it is positioned on both sides of the incision trace 6, illustrated by a broken line in Fig. 1. On one side of the incision trace a row of closing means 7 has been provided which cooperates with a row of closing means 8 arranged on the other side of the incision trace 6, one means 7i positioned on one side of the incision trace cooperating with a means 8i positioned oppositely on the other side of the incision trace. The closing means 7, 8 are designed in such a way that the two means 7i, 8i in a pair independently of the other pairs of mutually cooperating means may be brought into operative connection with each other for closing of the incision.

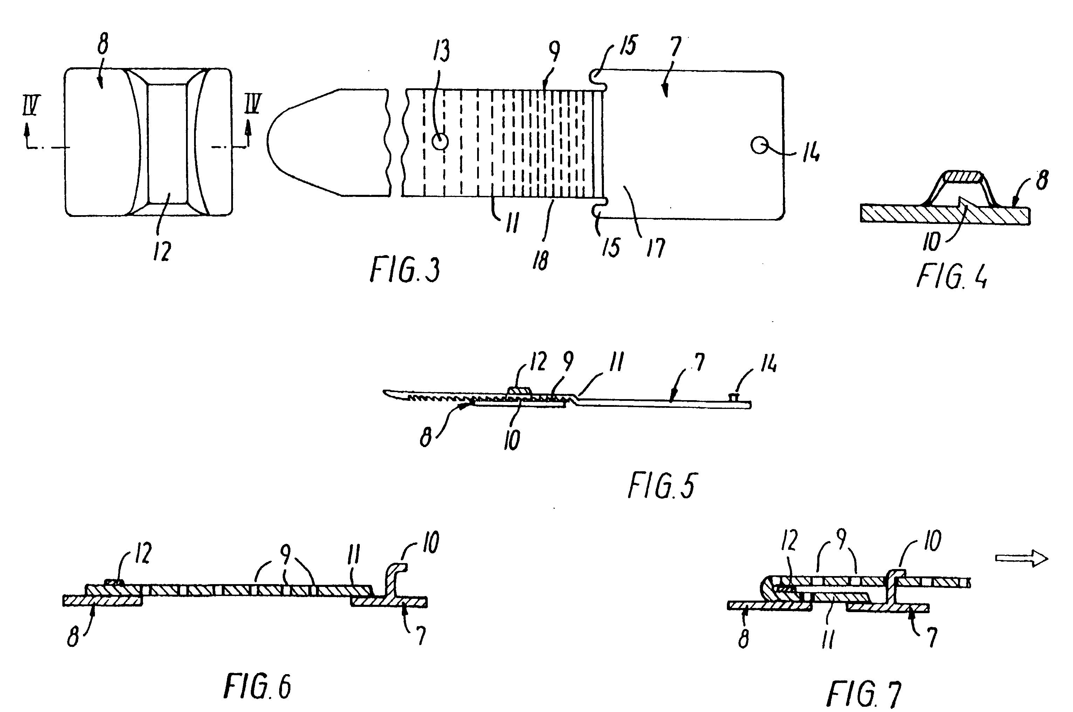

Further details of the closing means 7,8 shown in Fig. 1 appear from Figs. 3 and 4. As will be seen from Fig. 3 the closing means 7 is equipped with a strap-like element 11, the full length of which is not shown in Fig. 3. The closing means 8 is equipped with an eye- or loop-shaped guiding device 12, through which the strap-like element may be passed, following which it is then taken back in direction towards the closing means 7, such that a pull in the strap-like element 11 in this direction will subject the closing means 7, 8 to in a direction towards each other simultaneously acting incision closing forces substantially without any other impact on the area around the incision in the direction of the incision trace than what might be necessary for bringing the two means of a pair in line with each other. This pull may be exerted by one hand, which leaves the other hand of the surgeon free to bear against the body, which is not least of considerable importance when closing a longitudinal incision in a body in connection with autopsy.

The strap-like element 11 is on the side not shown equipped with locking means 9 in form of a row of transverse ribs 18 (indicated by broken lines), which when the strap-like element 11 is passed through the slot formed by the eye or loop 12 is brought into engagement with a catch protrusion 10, whereby the means 7, 8 are locked relative to each other. The ribs 18 do not have to be spaced with the same distance, but may for instance be placed with a shorter distance on the interior part, where the final tightening takes place. It may also just be a question of separate ribs arranged in predetermined locking positions. The locking may be released by a continued pull in the strap-like element 11. It is thus possible for the operator to make a controlled, gradual closing of the incision, the pairwise cooperating closing means 7, 8 being actuated alternately, and an already made, partial closing of the incision being maintained due to the locking of the ribs 18 relative to the ratchet protrusion 10.

The strap-like element 11 is provided with a hole 13 which when the strap-like element is bent backwards, away from the incision trace, may be brought into engagement with a tap 14 on the means 7. In this way the strap-like element 11 is safely kept away from the incision trace while the incision is being made. In the embodiment shown the means 7 is made from a material which is sufficiently resilient to make the transition between the fixed part 17 of the means 7 and the strap-like element act like a hinge. Nylon, polyethylene, and polypropylene are examples of such materials. In order to further protect the strap-like element against damage, while the incision is being made, the fixed part 17 of the means 7 is provided with a pair of protrusions 15 serving as guides for the scalpel, while the incision is being made, the backwards bent strap-like element 11 being protected at the same time.

Fig. 5 is a lateral view of the closing means according to Fig. 3 in closed condition. If desired, the strap-like element may be shortened after the closing has taken place.

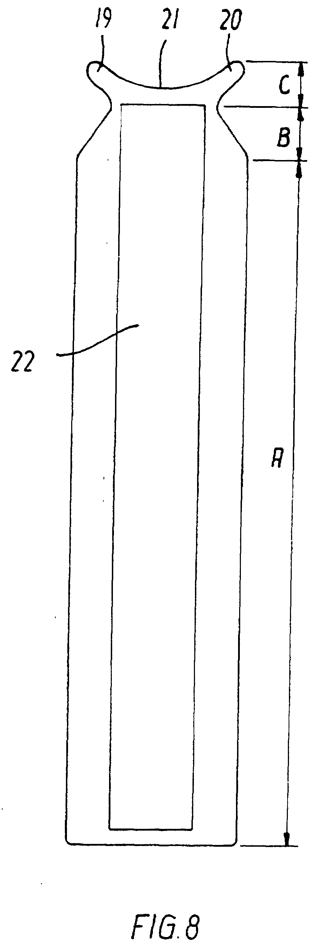

Fig. 6 and 7 illustrate an alternative embodiment of the closing means according to the invention, elements having the same function as elements shown in Figs. 1-5 being indicated by the same reference numerals for the sake of simplicity. In this embodiment the means for releasable closing of the closing means of a pair are a row of holes 9 arranged in the strap-like element 11, which, as shown in Fig. 7, is brought into engagement with a tap 10 on the means 7 with the strap-like element, the strap-like element having been passed through an eye or loop 12 on the means 8. The locking may be released by a continued pull in the strap-like element 11 in the direction of the arrow shown in Fig. 7. The holes 9 and the tap 10 may optionally be designed in such manner that they may both be used for keeping the strap-like element 11 free of the incision trace, while the incision is being made, and for locking of the means 7, 8 relative to each other after the passing of the strap-like element 11 around the eye or loop 12.

Fig. 8 shows the device according to Fig. 1 but after it has been used for closing an incision tracewise of a body in connection with autopsy. It will be seen how the incision line and the closing means which have been used for closing the incision are covered by a further sheet of material 22 adhesively attached to the device 1. It is moreover seen how the device 1 comprises a substantially rectangular elongate portion A which at one end merges into a portion B having side lines converging towards the centre line, which portion ends in a neck portion C with an obliquely outwards directed flap portion 19, 20 on each side of the centre line. Furthermore, a indentation 21 is provided in the neck portion C for receiving the curvature of a neck.

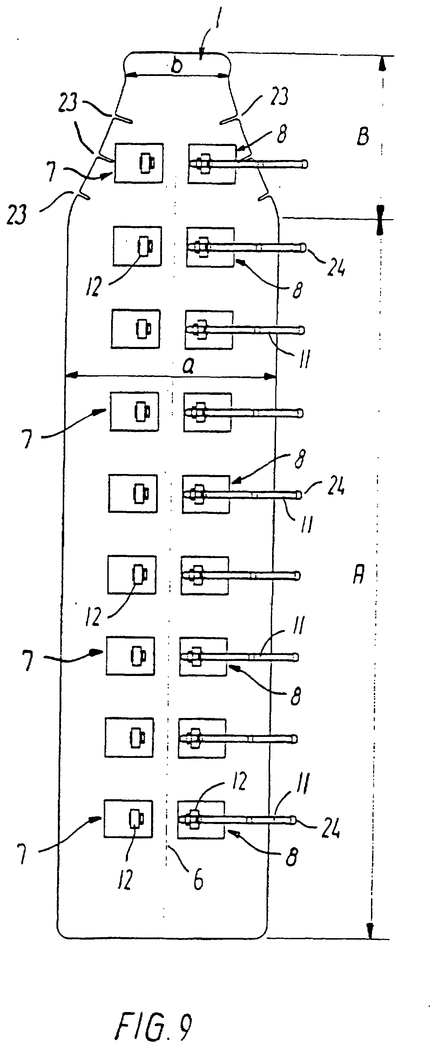

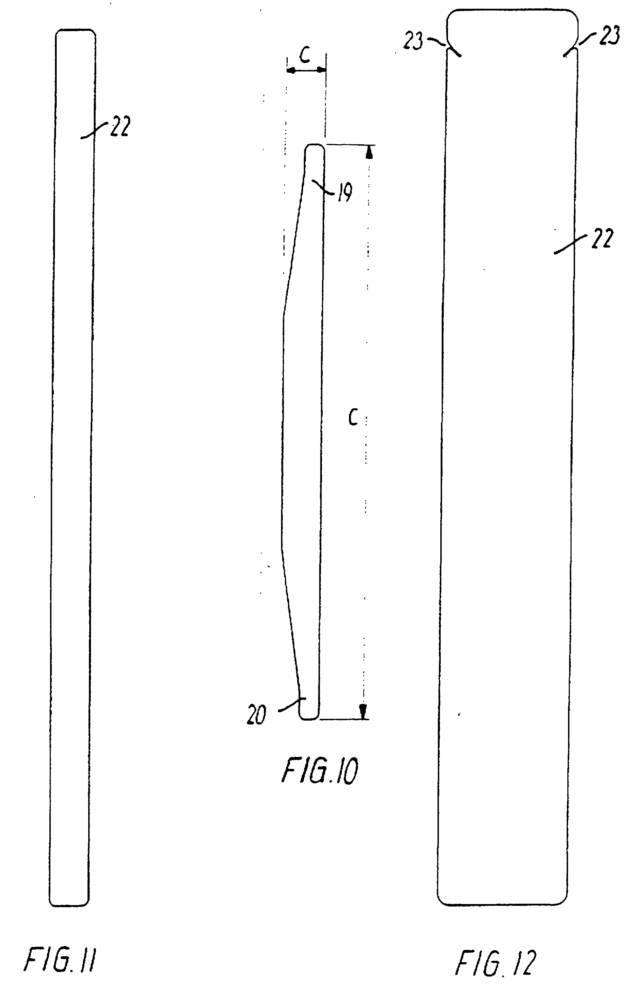

Figs. 9 and 10 show in combination another embodiment of the device according to the invention, in which the neck portion C is formed as a separate unit. As will be seen from Fig. 10 the neck portion C is substantially trapezoidal, as it has the shape of a trapezium, the corners of which at the base line of the trapezium being cut off and replaced by flap portions 19, 20 having a width of 1 - 2 cm.

From Fig. 9 will be seen how the device 1 comprises a substantially rectangular elongate portion A which at one end merges into a portion B with side lines converging towards the centre line.

The ratio between the distance c, spanned by the flap portions 19, 20 and the intermediate part of the neck portion, and the width b of the portion B in Fig. 9, where the distance between the substantially converging side lines is smallest, is in the embodiment shown about 5.5, and the ratio between the width a of the portion A and the above-mentioned width b is about 2.

The device 1 shown in Fig. 9 is in principle constructed in the same way as the device shown in Fig. 1, but the closing means are of another type. Thus both the closing means 7 and the closing means 8 comprise a guiding device 12 of the type shown in Figs. 3 and 5, whereas the strap-like element 11 is formed as a separate unit intended to be passed through both the guiding device 12 on the closing means 8 and the guiding device 12 on the closing means 7. To prevent the strap-like element 11 from passing all the way through both guiding devices 12 it is at one end provided with a stop member 24. In the same way as in case of the strap-like element shown in Fig. 3, the strap-like element 11 is provided with one or more ribs, by means of which it may be brought into locking engagement with the guiding device 12 on the opposite side of the incision trace 6. The closing means 7, 8 are designed with a comparatively large base to prevent delamination of the subjacent skin during the considerable impact of force occurring during the closing operation. The closing means are moreover arranged with a certain mutual spacing in a direction transversal to the incision trace, which does not only make it possible to tighten the tissue to the position, in which it was prior to the making of the incision, but to perform a further tightening, whereby the incision edges with the adhesively attached, flexible sheet of material and the subjacent skin are bent down into the incision area, and the upper surface of the edge areas of the sheet of material on each side of the incision trace abut each other, thereby contributing to an improved sealing. With a view to this, the edge area of the closing means 7, 8 facing the incision trace has been bevelled to facilitate the bending down in the incision area.

The edges running along the side lines of the portion B are provided with notches or indentations 23 for facilitating the adaptation to the contour of the body in the area in question. The notches or the indentations have been made by removal of a part of the sheet of material to reduce the risk of unintentional sticking together of the edges.

Figs. 11 and 12 show additional flexible sheets of material 22, which are on one surface provided with a layer of a hydrocolloid-containing adhesive, which layer of adhesive is covered by a sheet of release paper or foil. The sheet of material shown in Fig. 11 is intended to be adhesively attached immediately over the incision area after closing of an incision by means of the device 1. The sheet of material shown in Fig. 12 is intended to be adhesively attached after the sheet of material shown in Fig. 11 and such that it covers said sheet of material and the closing means 7, 8. To facilitate the adaptation to the contour of the body the device shown in Fig. 12 is in a similar way as the device 1 provided with notches or indentations 23 in the side edge at the end intended to face the neck. The sheet of material shown in Fig. 11 is provided with a thicker adhesive layer than the sheet of material shown in Fig. 12, as the need for absorption of fluid is biggest in the incision area, and as it moreover may be desirable to obtain a certain filling effect in this area, partly on account of the incision, partly on account of the closing means.

The invention has been described above with special reference to closing of an incision in connection with autopsy. It will, however, be understood that the device according to the invention may also be used for other closing operations, for instance for closing an incision in connection with a surgical procedure.

It will, moreover, be evident for the person skilled in the art that the invention has been explained above by means of various concrete embodiments and that different modifications and variations may be made without deviating from the scope of the patent claims.

## Claims

1. A device (1) suited for use in closing an incision in a body in connection with autopsy, in particular a longitudinal incision along the centre line of the body, which device comprises at least one flexible sheet of material (2) with a surface (3) intended for facing the body and an opposite surface (4) intended for facing away from the body, the body facing surface being provided with an adhesive (5), by means of which the device may be adhesively attached to the body in such a way that the device is present on both sides of the incision trace (6), and which device comprises two rows of mutually cooperating means (7, 8) for use in closing the incision, said rows of closing means being present on each side of the incision trace (6) and allowing the incision to be made after adhesive attachment of the device, **characterized** in that said at least one sheet of material (2) comprises a portion (C) intended for being adhesively attached to the neck area of the body, which neck portion (C) comprises an outwardly extending flap portion (19, 20) on each side of a line corresponding to the centre line of the body, and in that said at least one sheet of material (2) at the end facing the neck portion (C) comprises a portion (B), the side lines of which in a direction towards the neck portion (C) substantially converge towards the line corresponding to the centre line of the body.

2. A device according to claim 1,
**characterized in that** the outwardly extending flap portions (19, 20) have such a length that they together with the intermediate part of the neck portion span a distance (c) of at least 25 cm, preferably at least 35 cm and particularly a distance of 40 - 55 cm.

3. A device according to claim 1 or 2,
**characterized in that** the ratio between the distance (c), which the outwardly extending flap portions (19, 20) and the intermediate part of the neck portion span, and the width (b) of the portion (B), where the distance between the substantially converging side lines is smallest, is about 1.5 or more.

4. A device according one or more of the preceding claims, **characterized in that** the flap portions (19, 20) extend obliquely outwards and upwards seen relative to a line corresponding to the centre line of the body and relative to the orientation of the body in upright position, respectively.

5. A device according to one or more of the preceding claims, **characterized in that** the neck portion (C) is formed as a separate unit.

6. A device according to one or more of the preceding claims, **characterized in that** the neck portion (C) is substantially trapezoidal.

7. A device according to one or more of the preceding claims, **characterized in that** the neck portion (C) is provided with a indentation (21) for receiving the curvature of a neck.

8. A device according to claim 6,
**characterized in that** the edges running along the side lines of portion (B) are provided with notches or indentations (23).

9. A device according to claim 8,
**characterized in that** the notches or the indentations (23) are formed removing a part of the sheet of material (2).

10. A device according to one or more of the preceding claims, **characterized in that** said at least one sheet of material (2) at the end facing away from the neck portion (C) comprises a substantially rectangular oblong portion (A).

11. A device according to claim 10,
**characterized in that** the ratio between the width (a) of the portion (A) and the width (b) of the portion (B), where the distance between the substantially converging side lines is smallest, is about 1.2 or more, preferably about 1.5 or more and in particular 1.5 to 2.5.

12. A device according to one or more of the preceding claims, **characterized in that** the closing means (7, 8) cooperate in pairs, one means (7i) positioned on one side of the incision trace cooperating with a means (8i) positioned oppositely on the other side of the incision trace, and the two means of a pair independently of the remaining pairs of cooperating means may be connected operatively for closing the incision.

13. A device according to claim 12, **characterized in that** the closing means of a pair is provided with means (9, 10) for releasable locking of said closing means relative to each other during the closing operation.

14. A device according to claim 12 or 13, **characterized in that** one means of a pair comprises a strap-like element (11), and the other means of a pair comprises a guiding device (12) around or through which the strap-like element may be passed, such that a pull in the strap-like element will be able to produce an incision closing force substantially without producing any other impact on the area around the incision in the direction of the incision trace than what might be necessary for bringing the two means of a pair in line with each other.

15. A device according to claim 14, **characterized in that** the closing means with the strap-like element is provided with means (13, 14) for keeping the strap-like element away from the incision trace during the making of the incision.

16. A device according to claim 15,
**characterized in that** the means (13, 14) for keeping the strap-like element away from the incision trace during the making of the incision have the form of a tap-tap-hole arrangement, a button-buttonhole arrangement, a clamping device, a snap fastener, a strap, an adhesive tape, an adhesive and/or "Velcro".

17. A device according to claim 14
**characterized in that** the closing means with the strap-like element (11) is provided with means (15) for keeping the incision tool away from the strap-like element during the making of the incision.

18. A device according to one or more of the preceding claims, **characterized in that** the closing means (7,8) are arranged with a mutual spacing of at least 15 mm, preferably at least 20 mm in a direction transversely to the incision trace.

19. A device according to one or more of the preceding claims, **characterized in that** the width of the base of the closing means (7, 8) is at least 15 mm, preferably at least 20 mm and in particular at least 25 mm, and **in that** the length of the base of the closing means (7, 8) is at least 15 mm, preferably at least 20 mm and in particular at least 25 mm.

20. A device according to one or more of the preceding claims, **characterized in that** the edge area of the base of the closing means (7, 8) facing the incision trace is flexible.

21. A device according to one or more of the preceding claims, **characterized in that** one of the closing means (7, 8) comprises a strap-like element (11), which is provided with at least two ribs or cams (18), one of which corresponds to a fully closed position and the other one corresponds to an intermediate position, and **in that** the other of the closing means (7, 8) comprises a catch buckle.

22. A device according to claim 21,
**characterized in that** the strap-like element and the catch buckle are fastened at least 2 mm away from edge of the base of the closing means facing the incision trace.

23. A device according to one or more of the preceding claims, **characterized in that** the mutually co-operating means (7, 8) comprise guiding devices (12) which are positioned on either side of the incision trace, and separate, strap-like elements (11), by means of which the mutually cooperating means (7, 8) pairwise may be brought in operative connection with each other for closing the incision, said strap-like elements (11) being provided with a stop member (24) adapted to prevent the strap-like element from passing transversely through the guiding device (12) on one side of the incision trace and means for bringing it into locking engagement with the guiding device (12) on the other side of the incision trace in at least one position.

24. A device according to claims 12-23 **characterized in that** the closing means (7, 8) are designed in such a way that when the two means (7i, 8i) of a pair have been brought into operative connection with each other it is possible by a one hand pull in one of the means of the pair to apply to both means of the pair in a direction towards each other simultaneously acting incision closing forces substantially without any other impact on the area around the incision in the direction of the incision trace than what might be necessary for bringing, the two means of the pair in line with each other.

25. A device according to claims 13-24, **characterized in that** the means (9, 10) for releasable locking of the means of a pair are of catch buckle, belt buckle, hook buckle, button-buttonhole, tap-taphole, snap fastener or "Velcro" type.

26. A device according to claims 13-25 **characterized in that** the means (9, 10) for releasable locking of the closing means of a pair are positioned on the closing means with the strap-like element (11).

27. A device according to claims 13-26 **characterized in that** the means (9, 10) for releasable locking of the means of a pair are provided partly on one means of the pair and partly on the other.

28. A device according to claims 12-27, **characterized in that** the locking of the means of a pair relative to each other may be released substantially without any pulling force in a direction perpendicular to the plane of the sheet of material.

29. A device according to claim 28,
**characterized in that** the locking may be released by a continued pull in one of the means of the pair.

30. A device according to one or more of the preceding claims, **characterized in that** the sheet of material (2) is continuous in the area, in which the incision is to be made.

31. A device according to one or more of the preceding claims, **characterized in that** the sheet of material (2) is a foil of polyurethane, EVA (ethylene vinyl acetate) with a high content of vinyl acetate, chlorinated polyethylene or plasticized PVC.

32. A device according to one or more of the preceding claims, **characterized in that** the adhesive (5) is a hydrocolloid-containing adhesive.

33. A device according to one or more of the preceding claims, **characterized in that** the adhesive on the body facing surface is covered by a sheet of release paper or foil (16).

34. A device according to one or more of the preceding claims, **characterized in that** the neck portion (C) is provided with two rows of mutually cooperating means (7, 8) extending into the outwardly directed flaps (19, 20) for use in closing an incision running transversely to the centre line of a body.

35. A set comprising a device according one or more of the preceding claims, as well as a flexible, preferably skin-coloured and preferably nontransparent further sheet of material (22) of such dimensions that after the closing of an incision by means of said device it may cover the incision area and preferably also the closing means (7, 8), a layer of adhesive having been applied to one surface of said further sheet of material, said adhesive layer being covered by a sheet of release paper or foil.

36. A set according to claim 35,
**characterized in** comprising two further sheets of material (22), the first one of which having such dimensions that after closing of an incision by means of said device it may cover the incision area, and the other one having such dimensions that after closing of an incision by means of said device and after the first further sheet of material having been adhered over the incision area it may cover the closing means (7, 8).

37. A set according to claim 35 or 36,
**characterized in that** at least one of the further sheets of material (22) has been provided with an adhesive as stated in claim 32.

38. A set according to one or more of the claims 35 - 37, **characterized in that** the further sheet of material (22) or at least one of the further sheets of material (22) immediately above the incision area has been provided with an adhesive layer, which is thicker, preferably at least twice as thick and in particular at least 3 - 5 times as thick as the adhesive layer on the device itself.

39. A set according to one or more of the claims 35 - 38, **characterized in that** it comprises a further sheet of material (22) of such dimensions that it after closing of an incision by means of the device (1) substantially may cover the incision area and the closing means, said further sheet of material having rounded corners at the end intended for facing the neck and being at this end provided with notches or indentations (23) in the side edge.

40. A set according to claim 39,
**characterized in that** the notches or the indentations (23) are formed removing a part of the sheet of material (2).

41. A method for closing an incision in a body in connection with autopsy, in particular a longitudinal incision along the centre line of the body,
**characterized in that** before making the incision a device (1) according to one or more of the claims 1 - 34 is adhesively attached to the body in such a way that the device is positioned on both sides of the intended incision trace (6), the two rows of mutually cooperating means (7, 8) being positioned on either side of the incision trace and the neck portion (C) being adhesively attached in the neck area of the body with the outwardly extending flaps (19, 20) placed around the neck, possibly in such a way that they overlap, that after the making of the incision the mutually cooperating means are brought into operative connection with each other for closing the incision, the means cooperating in pairs being, if desired, operated alternately for a controlled gradual closing of the incision under releasable locking in at least one intermediate position, and the locking operation being, if desired, continued beyond the starting position such that the incision edges with the adhesively attached sheet of material and the subjacent skin bend downwardly into the incision area and such that the edge areas of the sheet of the material on either side of the incision trace abut, and **in that**, if desired, after possible wiping of the upper side of the device, one or more further sheets of material (22) as stated in claims 35 - 40 are adhesively attached for covering the incision area and preferably also the closing means (7, 8).

## Patentansprüche

1. Vorrichtung (1), die zur Verwendung beim Schließen eines Schnittes in einem Körper in Verbindung mit Autopsie und insbesondere eines Längsschnittes entlang der Zentrallinie des Körpers geeignet ist, wobei die Vorrichtung zumindest einen flexiblen Materialbogen (2) mit einer Oberfläche (3), die dazu bestimmt ist, zu dem Körper zu weisen, und einer gegenüberliegenden Oberfläche (4) umfasst, die dazu bestimmt ist, von dem Körper wegzuweisen, wobei die zu dem Körper weisende Oberfläche mit einem Klebstoff (5) versehen ist, mit dem die Vorrichtung an dem Körper derart angeklebt werden kann, dass die Vorrichtung auf beiden Seiten der Schnittspur (6) vorhanden ist, und wobei die Vorrichtung zwei Reihen miteinander zusammenwirkender Mittel (7, 8) zum Gebrauch zum Schließen des Schnittes umfasst, wobei die Reihen an Verschlussmitteln auf jeder Seite der Schnittspur (6) vorhanden sind und ermöglichen, dass der Schnitt nach einer Klebebefestigung der Vorrichtung ausgeführt werden kann, **dadurch gekennzeichnet, dass** der zumindest eine Materialbogen (2) einen Abschnitt (C) umfasst, der dazu bestimmt ist, an dem Halsbereich des Körpers angeklebt zu werden, wobei der Halsabschnitt (C) einen sich auswärts erstreckenden Klappenabschnitt (19, 20) auf jeder Seite einer Linie entsprechend der Zentrallinie des Körpers umfasst, und dass der zumindest eine Materialbogen (2) an dem Ende, das zu dem Halsabschnitt (C) weist, einen Abschnitt (B) umfasst, dessen Seitenlinien in einer Richtung zu dem Halsabschnitt (C) im wesentlichen in Richtung der Linie entsprechend der Zentrallinie des Körpers konvergieren.

2. Vorrichtung nach Anspruch 1,
**dadurch gekennzeichnet, dass**
die sich auswärts erstreckenden Klappenabschnitte (19, 20) eine solche Länge aufweisen, dass sie zusammen mit dem Zwischenteil des Halsabschnittes eine Distanz (c) von zumindest 25 cm, vorzugsweise zumindest 35 cm und insbesondere eine Distanz von 40 - 55 cm überspannen.

3. Vorrichtung nach einem der Ansprüche 1 oder 2,
**dadurch gekennzeichnet, dass**
das Verhältnis zwischen der Distanz (c), die von den sich auswärts erstreckenden Klappenabschnitten (19, 20) und dem Zwischenteil des Halsabschnittes überspannt wird, und der Breite (b) des Abschnittes (B), wo die Distanz zwischen den im wesentlichen konvergierenden Seitenlinien am kleinsten ist, etwa 1,5 oder mehr beträgt.

4. Vorrichtung nach einem der vorhergehenden Ansprüche,
**dadurch gekennzeichnet, dass**
sich die Klappenabschnitte (19, 20) schräg auswärts und aufwärts relativ zu einer Linie entsprechend der Zentrallinie des Körpers bzw. relativ zu der Orientierung des Körpers in aufrechter Stellung gesehen erstrecken.

5. Vorrichtung nach einem der vorhergehenden Ansprüche,
**dadurch gekennzeichnet, dass**
der Halsabschnitt (C) als eine separate Einheit ausgebildet ist.

6. Vorrichtung nach einem der vorhergehenden Ansprüche,
**dadurch gekennzeichnet, dass**
der Halsabschnitt (C) im Wesentlichen trapezförmig ist.

7. Vorrichtung nach einem der vorhergehenden Ansprüche,
**dadurch gekennzeichnet, dass** der Halsabschnitt (C) mit einer Vertiefung (21) zur Aufnahme der Krümmung eines Halses versehen ist.

8. Vorrichtung nach Anspruch 6,
**dadurch gekennzeichnet, dass** die Ränder, die entlang der Seitenlinien des Abschnittes (B) verlaufen, mit Kerben oder Vertiefungen (23) versehen sind.

9. Vorrichtung nach Anspruch 8,
**dadurch gekennzeichnet, dass**
die Kerben oder die Vertiefungen (23) durch Entfernen eines Teils des Materialbogens (2) gebildet werden.

10. Vorrichtung nach einem der vorhergehenden Ansprüche,
**dadurch gekennzeichnet, dass**
der zumindest eine Materialbogen (2) an dem Ende, das von dem Halsabschnitt (C) wegweist, einen im Wesentlichen rechteckigen länglichen Abschnitt (A) umfasst.

11. Vorrichtung nach Anspruch 10,
**dadurch gekennzeichnet, dass**
das Verhältnis zwischen der Breite (a) des Abschnittes (A) und der Breite (b) des Abschnittes (B), wo die Distanz zwischen den im wesentlichen konvergierenden Seitenlinien am kleinsten ist, etwa 1,2 oder mehr, vorzugsweise etwa 1,5 oder mehr und insbesondere 1,5 bis 2,5 beträgt.

12. Vorrichtung nach einem der vorhergehenden Ansprüche,
**dadurch gekennzeichnet, dass**
die Verschlussmittel (7, 8) in Paaren zusammenwirken, wobei ein Mittel (7i), das auf einer Seite der Schnittspur positioniert ist, mit einem Mittel (8i) zusammenwirkt, das gegenüberliegend auf der anderen Seite der Schnittspur positioniert ist, und die beiden Mittel eines Paares unabhängig von den verbleibenden Paaren an zusammenwirkenden Mitteln wirksam zum Schließen des Schnittes verbunden werden können.

13. Vorrichtung nach Anspruch 12,
**dadurch gekennzeichnet, dass**
das Verschlussmittel eines Paares mit Mitteln (9, 10) versehen ist, um die Verschlussmittel relativ zueinander während des Verschlussvorganges freigebbar zu verriegeln.

14. Vorrichtung nach Anspruch 12 oder 13,
**dadurch gekennzeichnet, dass**
ein Mittel eines Paares ein riemenartiges Element (11) umfasst, und das andere Mittel eines Paares eine Führungsvorrichtung (12) umfasst, um oder durch die das riemenartige Element geführt werden kann, so dass ein Ziehen an dem riemenartigen Element zur Folge hat, dass eine Kraft zum Schließen eines Schnittes erzeugt wird, im Wesentlichen ohne dass eine andere Einwirkung auf den Bereich um den Schnitt in der Richtung der Schnittspur erzeugt wird, als es erforderlich wäre, um die beiden Mittel eines Paares in Linie zueinander zu bringen.

15. Vorrichtung nach Anspruch 14,
**dadurch gekennzeichnet, dass**
das Verschlussmittel mit dem riemenartigen Element mit Mitteln (13, 14) versehen ist, um das riemenartige Element während der Ausbildung des Schnittes weg von der Schnittspur zu halten.

16. Vorrichtung nach Anspruch 15,
**dadurch gekennzeichnet, dass**
die Mittel (13, 14), um das riemenartige Element weg von der Schnittspur während der Ausbildung des Schnittes zu halten, die Form einer Anordnung aus Dorn und Stichloch, einer Anordnung aus Knopf und Knopfloch, einer Klemmvorrichtung, einer Schnappbefestigungseinrichtung, eines Riemens, eines Klebebandes, eines Klebstoffes und/oder "Velcro" (Klettverschluss) aufweist.

17. Vorrichtung nach Anspruch 14,
**dadurch gekennzeichnet, dass**
das Verschlussmittel mit dem riemenartigen Element (11) mit Mitteln 15 versehen ist, um das Schnittwerkzeug weg von dem riemenartigen Element während der Ausbildung des Schnittes zu halten.

18. Vorrichtung nach einem der vorhergehenden Ansprüche,
**dadurch gekennzeichnet, dass**
die Verschlussmittel (7, 8) mit einem gegenseitigen Abstand von zumindest 15 mm und vorzugsweise zumindest 20 mm in einer Richtung quer zu der Schnittspur angeordnet sind.

19. Vorrichtung nach einem der vorhergehenden Ansprüche,
**dadurch gekennzeichnet, dass**
die Breite der Basis des Verschlussmittels (7, 8) zumindest 15 mm, vorzugsweise zumindest 20 mm und insbesondere zumindest 25 mm beträgt, und dass die Länge der Basis des Verschlussmittels (7, 8) zumindest 15 mm, vorzugsweise zumindest 20 mm und insbesondere zumindest 25 mm beträgt.

20. Vorrichtung nach einem der vorhergehenden Ansprüche,
**dadurch gekennzeichnet, dass**
der Randbereich der Basis des Verschlussmittels (7, 8), der zu der Schnittspur weist, flexibel ist.

21. Vorrichtung nach einem der vorhergehenden Ansprüche,
**dadurch gekennzeichnet, dass**
eines der Verschlussmittel (7, 8) ein riemenartiges Element (11) umfasst, das mit zumindest zwei Rippen oder Nocken (18) versehen ist, von denen eine einer vollständig geschlossenen Position und die andere einer Zwischenposition entspricht, und dass das andere der Verschlussmittel (7, 8) eine Rastschnalle umfasst.

22. Vorrichtung nach Anspruch 21,
**dadurch gekennzeichnet, dass**
das riemenartige Element und die Rastschnalle zumindest 2 mm entfernt von dem Rand der Basis des Verschlussmittels, der zu der Schnittspur weist, befestigt sind.

23. Vorrichtung nach einem der vorhergehenden Ansprüche,
**dadurch gekennzeichnet, dass**
die miteinander zusammenwirkenden Mittel (7, 8) Führungsvorrichtungen (12), die auf jeder Seite der Schnittspur positioniert sind, und separate riemenartige Elemente (11) umfassen, mit denen die miteinander zusammenwirkenden Mittel (7, 8) paarweise in wirksame Verbindung miteinander gebracht werden können, um den Schnitt zu schließen, wobei die riemenartigen Elemente (11) mit einem Stoppelement (24), das dazu ausgebildet ist, um zu verhindern, dass das riemenartige Element quer durch die Führungsvorrichtung (12) auf einer Seite der Schnittspur läuft, und mit Mitteln versehen sind, um diese in Verriegelungseingriff mit der Führungsvorrichtung (12) auf der anderen Seite der Schnittspur in zumindest einer Position zu bringen.

24. Vorrichtung nach einem der Ansprüche 12 - 23,
**dadurch gekennzeichnet, dass**
die Verschlussmittel (7, 8) so ausgebildet sind, dass es, wenn die beiden Mittel (7i, 8i) eines Paares in wirksame Verbindung miteinander gebracht worden sind, möglich ist, durch Ziehen mit einer Hand an einem der Mittel des Paares an beide Mittel des Paares in einer Richtung zueinander simultan wirkende Kräfte zum Schließen eines Schnittes anzulegen, im Wesentlichen ohne dass eine andere Einwirkung auf den Bereich um den Schnitt herum in der Richtung der Schnittspur erfolgt, als es erforderlich wäre, um die beiden Mittel des Paares in Linie zueinander zu bringen.

25. Vorrichtung nach einem der Ansprüche 13 - 24,
**dadurch gekennzeichnet, dass**
die Mittel (9, 10) zur freigebbaren Verriegelung der Mittel eines Paares vom Typ mit Rastschnalle, Gurtschnalle, Hakenschnalle, Knopf und Knopfloch, Dorn und Stichloch, Schnappbefestigung oder "Velcro" (Klettverschluss) sind.

26. Vorrichtung nach einem der Ansprüche 13 - 25,
**dadurch gekennzeichnet, dass**
die Mittel (9, 10) zur freigebbaren Verriegelung der Verschlussmittel eines Paares an dem Verschlussmittel mit dem riemenartigen Element (11) positioniert sind.

27. Vorrichtung nach einem der Ansprüche 13 - 26,
**dadurch gekennzeichnet, dass**
die Mittel (9, 10) zur freigebbaren Verriegelung der Mittel eines Paares teilweise auf einem Mittel des Paares und teilweise auf dem anderen vorgesehen sind.

28. Vorrichtung nach einem der Ansprüche 12 - 27,
**dadurch gekennzeichnet, dass**
die Verriegelung der Mittel eines Paares relativ zueinander im Wesentlichen ohne irgendeine Zugkraft in einer Richtung rechtwinklig zu der Ebene des Materialbogens freigegeben werden kann.

29. Vorrichtung nach Anspruch 28,
**dadurch gekennzeichnet, dass**
die Verriegelung durch ein kontinuierliches Ziehen an einem der Mittel des Paares freigegeben werden kann.

30. Vorrichtung nach einem der vorhergehenden Ansprüche,
**dadurch gekennzeichnet, dass**
der Materialbogen (2) in dem Bereich kontinuierlich ist, in dem der Schnitt ausgeführt wird.

31. Vorrichtung nach einem der vorhergehenden Ansprüche,
**dadurch gekennzeichnet, dass**
der Materialbogen (2) aus einer Folie aus Polyurethan, EVA (Ethylenvinylacetat) mit einem hohen Gehalt an Vinylacetat, chloriertem Polyethylen oder Weich-PVC besteht.

32. Vorrichtung nach einem der vorhergehenden Ansprüche,
**dadurch gekennzeichnet, dass**
der Klebstoff (5) ein hydrokolloidhaltiger Klebstoff ist.

33. Vorrichtung nach einem der vorhergehenden Ansprüche,
**dadurch gekennzeichnet, dass**
der Klebstoff an der zu dem Körper weisenden Oberfläche mit einem Bogen an Schutz- / Abziehpapier oder -folie (16) bedeckt ist.

34. Vorrichtung nach einem der vorhergehenden Ansprüche,
**dadurch gekennzeichnet, dass**
der Halsabschnitt (C) mit zwei Reihen miteinander zusammenwirkender Mittel (7, 8) versehen ist, die sich in die auswärtsgerichteten Klappen (19, 20) zum Gebrauch beim Schließen eines Schnittes erstrecken, der quer zu der Zentrallinie eines Körpers verläuft.

35. Satz mit einer Vorrichtung gemäß einem der vorhergehenden Ansprüche, wie auch einem flexiblen, vorzugsweise hautfarbenen und vorzugsweise nicht transparenten weiteren Materialbogen (22) mit solchen Abmessungen, dass nach dem Schließen eines Schnittes mittels der Vorrichtung dieser den Schnittbereich und vorzugsweise auch die Verschlussmittel (7, 8) bedecken kann, wobei eine Klebstofflage auf eine Oberfläche des weiteren Materialbogens aufgetragen worden ist, wobei die Klebstofflage mit einem Bogen an Schutz- / Abziehpapier oder -folie bedeckt ist.

36. Satz nach Anspruch 35,
**dadurch gekennzeichnet, dass**
ferner zwei weitere Materialbögen (22) vorgesehen sind, wobei der erste derartige Abmessungen aufweist, dass er nach einem Schließen eines Schnittes mittels der Vorrichtung den Schnittbereich bedecken kann, und der andere derartige Abmessungen aufweist, dass er nach einem Schließen eines Schnittes mittels der Vorrichtung und nachdem der erste weitere Materialbogen über den Schnittbereich angeklebt worden ist, die Verschlussmittel (7, 8) bedecken kann.

37. Satz nach Anspruch 35 oder 36,
**dadurch gekennzeichnet, dass**
der zumindest eine der weiteren Materialbögen (22) mit einem Klebstoff versehen worden ist, wie in Anspruch 32 angegeben ist.

38. Satz nach einem der vorhergehenden Ansprüche 35 - 37, **dadurch gekennzeichnet, dass**
der weitere Materialbogen (22) oder zumindest einer der weiteren Materialbögen (22) unmittelbar über dem Schnittbereich mit einer Klebstofflage versehen worden ist, die dicker, vorzugsweise zumindest doppelt so dick und insbesondere zumindest 3 - 5 mal so dick wie die Klebstofflage an der Vorrichtung selbst ist.

39. Satz nach einem der vorhergehenden Ansprüche 35 - 38,
**dadurch gekennzeichnet, dass**
dieser einen weiteren Materialbogen (22) mit solchen Abmessungen umfasst, dass er nach einem Schließen eines Schnittes mittels der Vorrichtung (1) den Schnittbereich und die Verschlussmittel im Wesentlichen bedecken kann, wobei der weitere Materialbogen abgerundete Ecken an dem Ende aufweist, das dazu bestimmt ist, zu dem Hals zu weisen, und an diesem Ende mit Kerben oder Vertiefungen (23) in dem Seitenrand versehen ist.

40. Satz nach Anspruch 39,
**dadurch gekennzeichnet, dass**
die Kerben oder die Vertiefungen (23) durch Entfernung eines Teiles des Materialbogens (2) ausgebildet sind.

41. Verfahren zum Schließen eines Schnittes in einem Körper in Verbindung mit Autopsie, insbesondere eines Längsschnittes entlang der Zentrallinie des Körpers,
**dadurch gekennzeichnet, dass**
vor der Ausbildung des Schnittes eine Vorrichtung (1) gemäß einem der Ansprüche 1 - 34 an den Körper so geklebt wird, dass die Vorrichtung auf beiden Seiten der beabsichtigten Schnittspur (6) positioniert ist, wobei die beiden Reihen miteinander zusammenwirkender Mittel (7, 8) auf jeder Seite der Schnittspur positioniert sind, und der Halsabschnitt (C) in dem Halsbereich des Körpers angeklebt ist, wobei die sich auswärts erstreckenden Klappen (19, 20) um den Hals möglicherweise in einer solchen Art und Weise angeordnet sind, dass sie sich überlappen, dass nach der Ausbildung des Schnittes die miteinander zusammenwirkenden Mittel in wirksame Verbindung miteinander gebracht werden, um den Schnitt zu schließen, wobei die Mittel, die in Paaren zusammenwirken, gegebenenfalls abwechselnd für ein gesteuertes allmähliches Schließen des Schnittes unter freigebbarem Verriegeln in zumindest eine Zwischenposition betätigt werden, und sich der Verriegelungsvorgang gegebenenfalls über die Startposition hinaus fortsetzt, so dass die Schnittränder mit dem angeklebten Materialbogen und die darunterliegende Haut abwärts in den Schnittbereich gebogen werden, so dass die Randbereiche des Materialbogens auf jeder Seite der Schnittspur aneinander anstoßen, und dass gegebenenfalls nach einem möglichen Überstreifen der oberen Seite der Vorrichtung eine oder mehrere weitere Materialbögen (22), wie in den Ansprüchen 35 - 40 angegeben ist, zur Bedeckung des Schnittbereiches und vorzugsweise auch der Verschlussmittel (7, 8) angeklebt werden.

## Revendications

1. Un dispositif (1) pouvant être utilisé pour la fermeture d'une incision pratiquée dans un corps en rapport avec une autopsie, en particulier une incision longitudinale le long de la ligne médiane du corps, lequel dispositif comprend au moins une feuille flexible de matériau (2) avec une face (3) destinée à être tournée vers le corps et une face opposée (4) destinée à être tournée vers l'extérieur du corps, la face tournée vers le corps étant fournie avec un adhésif (5), au moyen duquel le dispositif peut être fixé au corps de manière adhésive de telle manière que le dispositif soit présent des deux côtés de la ligne d'incision (6), et lequel dispositif comprend deux rangées de moyens fonctionnant de manière complémentaire (7, 8) devant servir à fermer l'incision, lesdites rangées de moyens de fermeture étant présentes de chaque côté de la ligne d'incision (6) et permettant de pratiquer l'incision après la fixation adhésive du dispositif, ledit dispositif étant **caractérisé par le fait que** ladite au moins une feuille de matériau (2) comprend une portion (C) destinée à être fixée de manière adhésive à la zone du cou du corps, laquelle portion destinée au cou (C) comprend une portion en volet s'étendant vers l'extérieur (19, 20) de chaque côté d'une ligne correspondant à la ligne médiane du corps, et **par le fait que** ladite au moins une feuille de matériau (2) à l'extrémité faisant face à la portion destinée au cou (C) comprend une portion (B), dont les lignes latérales en direction de la portion destinée au cou (C) convergent de essentiellement vers la ligne correspondant à la ligne médiane du corps.

2. Un dispositif tel que revendiqué dans la revendication 1,
**caractérisé par le fait que** les portions en volet s'étendant vers l'extérieur (19, 20) ont une longueur telle qu'avec la partie intermédiaire de la portion destinée au cou, elles couvrent une distance (c) d'au moins 25 cm, de préférence d'au moins 35 cm, et en particulier une distance de 40-55 cm.

3. Un dispositif tel que revendiqué dans la revendication 1 ou 2,
**caractérisé par le fait que** le rapport entre la distance (c), que couvrent les portions en volet s'étendant vers l'extérieur (19, 20) avec la partie intermédiaire de la portion destinée au cou, et la largeur (b) de la portion (B), là où la distance entre les lignes latérales essentiellement convergentes est la plus petite, est d'environ 1,5 ou plus.

4. Un dispositif tel que revendiqué dans une ou plusieurs des revendications précédentes, **caractérisé par le fait que** les portions en volet (19, 20) s'étendent à l'oblique vers l'extérieur et vers le haut, respectivement par rapport à une ligne correspondant à la ligne médiane du corps et par rapport à l'orientation du corps placé en position droite.

5. Un dispositif tel que revendiqué dans une ou plusieurs des revendications précédentes, **caractérisé par le fait que** la portion destinée au cou (C) est réalisée sous forme d'une unité séparée.

6. Un dispositif tel que revendiqué dans une ou plusieurs des revendications précédentes, **caractérisé par le fait que** la portion destinée au cou (C) est essentiellement trapézoïdale.

7. Un dispositif tel que revendiqué dans une ou plusieurs des revendications précédentes, **caractérisé par le fait que** la portion destinée au cou (C) est fournie avec une indentation (21) destinée à accueillir la courbure d'un cou.

8. Un dispositif tel que revendiqué dans la revendication 6,
**caractérisé par le fait que** les bords longeant les lignes latérales de la portion (B) sont fournis avec des encoches ou des indentations (23).

9. Un dispositif tel que revendiqué dans la revendication 8,
**caractérisé par le fait que** les encoches ou les indentations (23) sont réalisées en retirant une partie de la feuille de matériau (2).

10. Un dispositif tel que revendiqué dans une ou plusieurs des revendications précédentes, **caractérisé par le fait que** ladite au moins une feuille de matériau (2) à l'extrémité tournée à l'opposé de la portion destinée au cou (C) comprend une portion oblongue essentiellement rectangulaire (A).

11. Un dispositif tel que revendiqué dans la revendication 10,
**caractérisé par le fait que** le rapport entre la largeur (a) de la portion (A) et la largeur (b) de la portion (B), là où la distance entre les lignes latérales essentiellement convergeantes est la plus petite, est d'environ 1,2 ou plus, de préférence d'environ 1,5 ou plus, et en particulier de 1,5 à 2,5.

12. Un dispositif tel que revendiqué dans une ou plusieurs des revendications précédentes, **caractérisé par le fait que** les moyens de fermeture (7, 8) fonctionnent par paires, un moyen (7i) positionné d'un côté de la ligne d'incision fonctionnant avec un moyen (8i) positionné à l'opposé de l'autre côté de la ligne d'incision, et les deux moyens d'une même paire, indépendamment des autres paires de moyens de fermeture, peuvent être connectés de manière opérante pour fermer l'incision.

13. Un dispositif tel que revendiqué dans la revendication 12, **caractérisé par le fait que** le moyen de fermeture d'une paire est fourni avec des moyens (9, 10) pour déclencher le verrouillage dudit moyen de fermeture avec l'autre moyen au cours de l'opération de fermeture.

14. Un dispositif tel que revendiqué dans la revendication 12 ou 13, **caractérisé par le fait qu'**un moyen d'une paire comprend un élément de type courroie (11) et l'autre moyen d'une paire comprend un dispositif de guidage (12) autour ou au travers duquel l'élément de type courroie peut être passé, de manière à ce qu'une traction sur l'élément de type courroie soit capable de produire une force de fermeture de l'incision essentiellement sans produire tout autre impact sur la zone autour de l'incision dans la direction de la ligne d'incision que ce qui pourrait être nécessaire pour aligner les deux moyens d'une paire l'un avec l'autre.

15. Un dispositif tel que revendiqué dans la revendication 14, **caractérisé par le fait que** le moyen de fermeture avec l'élément de type courroie est fourni avec des moyens (13, 14) pour maintenir l'élément de type courroie à distance de la ligne d'incision lors de la réalisation de l'incision.

16. Un dispositif tel que revendiqué dans la revendication 15,
**caractérisé par le fait que** les moyens (13, 14) pour maintenir l'élément de type courroie à distance de la ligne d'incision lors de la réalisation de l'incision ont la forme d'un agencement de trou taraudé, d'un agencement de boutonnière, d'un dispositif de clampage, d'un bouton à pression, d'une courroie, d'un ruban adhésif, d'un adhésif et/ou d'un "Velcro".

17. Un dispositif tel que revendiqué dans la revendication 14, **caractérisé par le fait que** le moyen de fermeture avec l'élément de type courroie (11) est fourni avec des moyens (15) pour maintenir l'outil d'incision à distance de l'élément de type courroie lors de la réalisation de l'incision.

18. Un dispositif tel que revendiqué dans une ou plusieurs des revendications précédentes, **caractérisé par le fait que** les moyens de fermeture (7, 8) sont agencés avec un espacement mutuel d'au moins 15 mm, de préférence au moins 20 mm, dans une direction transversale par rapport à la ligne d'incision.

19. Un dispositif tel que revendiqué dans une ou plusieurs des revendications précédentes, **caractérisé par le fait que** la largeur de la base des moyens de fermeture (7, 8) est d'au moins 15 mm, de préférence d'au moins 20 mm, et en particulier d'au moins 25 mm, et **par le fait que** la longueur de la base des moyens de fermeture (7, 8) est d'au moins 15 mm, de préférence d'au moins 20 mm, et en particulier d'au moins 25 mm.

20. Un dispositif tel que revendiqué dans une ou plusieurs des revendications précédentes, **caractérisé par le fait que** le bord de la base des moyens de fermeture (7, 8) faisant face à la ligne d'incision est flexible.

21. Un dispositif tel que revendiqué dans une ou plusieurs des revendications précédentes, **caractérisé par le fait que** l'un des moyens de fermeture (7, 8) comprend un élément de type courroie (11), qui est fourni avec au moins deux nervures ou cames (18), dont l'une correspond à une position complètement fermée et l'autre correspond à une position intermédiaire, et **par le fait que** l'autre moyen de fermeture (7, 8) comprend une boucle à cliquet.

22. Un dispositif tel que revendiqué dans la revendication 21,
**caractérisé par le fait que** l'élément de type courroie et la boucle à cliquet sont fixés au moins à 2 mm de distance du bord de la base du moyen de fermeture faisant face à la ligne d'incision.

23. Un dispositif tel que revendiqué dans une ou plusieurs des revendications précédentes, **caractérisé par le fait que** les moyens fonctionnant de manière complémentaire (7, 8) comprennent des dispositifs de guidage (12), qui sont positionnés de chaque côté de la ligne d'incision, et des éléments de type courroie séparés (11), par l'intermédiaire desquels les moyens fonctionnant de manière complémentaire (7, 8) peuvent être connectés par paires de manière opérante l'un à l'autre afin de fermer l'incision, lesdits éléments de type courroie (11) étant fournis avec un membre d'arrêt (24) adapté pour empêcher l'élément de type courroie de passer à la transversale par le dispositif de guidage (12) d'un côté de la ligne d'incision et l'amener dans la prise de verrouillage avec le dispositif de guidage (12) de l'autre côté de la ligne d'incision dans au moins une position.

24. Un dispositif tel que revendiqué dans les revendications 12-23, **caractérisé par le fait que** les moyens de fermeture (7, 8) sont conçus de telle manière que lorsque les deux moyens (7i, 8i) d'une paire ont été connectés de manière opérante l'un à l'autre, il soit possible, en tirant d'une seule main sur l'un des moyens de la paire, d'appliquer aux deux moyens de la paire, dans une direction les rapprochant l'un de l'autre, des forces de fermeture de l'incision agissant simultanément, essentiellement sans produire tout autre impact sur la zone autour de l'incision dans la direction de la ligne d'incision que ce qui pourrait être nécessaire pour aligner les deux moyens d'une paire l'un avec l'autre.

25. Un dispositif tel que revendiqué dans les revendications 13-24, **caractérisé par le fait que** les moyens (9, 10) pour déclencher le verrouillage du moyen d'une paire sont de type boucle à cliquet, boucle à bande, boucle à crochet, boutonnière, trou taraudé, bouton à pression ou "Velcro".

26. Un dispositif tel que revendiqué dans les revendications 13-25, **caractérisé par le fait que** les moyens (9, 10) pour déclencher le verrouillage des moyens de fermeture d'une paire sont positionnés sur les moyens de fermeture avec l'élément de type courroie (11).

27. Un dispositif tel que revendiqué dans les revendications 13-26, **caractérisé par le fait que** les moyens (9, 10) pour déclencher le verrouillage des moyens d'une paire sont fournis en partie sur un moyen de la paire et en partie sur l'autre.

28. Un dispositif tel que revendiqué dans les revendications 12-27, **caractérisé par le fait que** le verrouillage des moyens d'une paire l'un avec l'autre peut être déclenché essentiellement sans aucune force de traction dans une direction perpendiculaire au plan de la feuille de matériau.

29. Un dispositif tel que revendiqué dans la revendication 28,
**caractérisé par le fait que** le verrouillage peut être déclenché par une traction continue sur l'un des moyens de la paire.

30. Un dispositif tel que revendiqué dans une ou plusieurs des revendications précédentes, **caractérisé par le fait que** la feuille de matériau (2) est continue dans la zone où l'incision doit être pratiquée.

31. Un dispositif tel que revendiqué dans une ou plusieurs des revendications précédentes, **caractérisé par le fait que** la feuille de matériau (2) est une feuille de polyuréthane, d'EAV (éthylène/acétate de vinyle), avec une teneur élevée en acétate de vinyle, en polyéthylène chloré ou en PVC plastifié.

32. Un dispositif tel que revendiqué dans une ou plusieurs des revendications précédentes, **caractérisé par le fait que** l'adhésif (5) est un adhésif contenant de l'hydrocolloïde.

33. Un dispositif tel que revendiqué dans une ou plusieurs des revendications précédentes, **caractérisé par le fait que** l'adhésif sur la face tournée vers le corps est couvert d'une feuille de papier ou film anti-adhésif.

34. Un dispositif tel que revendiqué dans une ou plusieurs des revendications précédentes, **caractérisé par le fait que** la portion destinée au cou (C) est fournie avec deux rangées de moyens fonctionnant de manière complémentaire (7, 8), s'étendant jusque dans les volets dirigés vers l'extérieur (19, 20) pour utilisation dans la fermeture d'une incision pratiquée à la transversale de la ligne médiane du corps.

35. Un ensemble comprenant un dispositif tel que revendiqué dans une ou plusieurs des revendications précédentes, ainsi qu'une feuille de matériau supplémentaire flexible, de préférence de la couleur de la peau et de préférence non transparente (22) de dimensions telles qu'après la fermeture d'une incision au moyen dudit dispositif, elle puisse recouvrir la zone de l'incision et de préférence également les moyens de fermeture (7, 8), une couche d'adhésif ayant été appliqué sur l'une des faces de ladite feuille de matériau supplémentaire, ladite couche adhésive étant recouverte d'une feuille de papier ou film anti-adhésif.

36. Un ensemble tel que revendiqué dans la revendication 35,
**caractérisé par le fait qu'**il comprend deux feuilles de matériau supplémentaires (22), la première d'entre elles ayant des dimensions telles qu'après la fermeture d'une incision au moyen dudit dispositif, elle puisse recouvrir la zone de l'incision, et la seconde ayant des dimensions telles qu'après la fermeture d'une incision au moyen dudit dispositif et après que la première feuille de matériau supplémentaire ait adhéré à l'ensemble de la zone de l'incision, elle puisse recouvrir les moyens de fermeture (7, 8).

37. Un ensemble tel que revendiqué dans la revendication 35 ou 36,
**caractérisé par le fait qu'**au moins une des feuilles de matériau supplémentaires (22) a été fournie avec un adhésif tel que revendiqué dans la revendication 32.

38. Un ensemble tel que revendiqué dans une ou plusieurs des revendications 35-37, **caractérisé par le fait que** la feuille de matériau supplémentaire (22) ou au moins l'une des feuilles de matériau supplémentaires (22), située immédiatement au-dessus de la zone de l'incision, a été fournie avec une couche adhésive, qui est plus épaisse, de préférence au moins deux fois plus épaisse, et en particulier au moins 3-5 fois plus épaisse que la couche adhésive sur le dispositif lui-même.

39. Un ensemble tel que revendiqué dans une ou plusieurs des revendications 35-38, **caractérisé par le fait qu'**il comprend une feuille de matériau supplémentaire (22) de dimensions telles qu'après fermeture d'une incision au moyen du dispositif (1), elle puisse essentiellement recouvrir la zone de l'incision et les moyens de fermeture, ladite feuille de matériau supplémentaire ayant des coins arrondis à l'extrémité destinée à faire face au cou et étant à cette fin équipée d'encoches ou d'indentations (23) sur le bord latéral.

40. Un ensemble tel que revendiqué dans la revendication 39,
**caractérisé par le fait que** les encoches ou les indentations (23) sont réalisées en retirant une partie de la feuille de matériau (2).

41. Une méthode de fermeture d'une incision pratiquée dans un corps en rapport avec une autopsie, en particulier une incision longitudinale le long de la ligne médiane du corps,
**caractérisée par le fait qu'**avant de pratiquer l'incision, un dispositif (1) tel que revendiqué dans une ou plusieurs des revendications 1-34 est fixé de manière adhésive au corps de telle manière que le dispositif soit positionné des deux côtés de la ligne d'incision prévue (6), les deux rangées de moyens fonctionnant de manière complémentaire (7, 8) étant positionnées de chaque côté de la ligne d'incision et la portion destinée au cou (C) étant fixée de manière adhésive à la zone du cou du corps avec les volets s'étendant vers l'extérieur (19, 20) placés autour du cou, éventuellement d'une manière telle qu'ils se chevauchent, qu'après la réalisation de l'incision, les moyens fonctionnant de manière complémentaire soient connectés de manière opérante l'un à l'autre pour fermer l'incision, les moyens fonctionnant en paires étant, si désiré, actionnés l'un après l'autre pour permettre une fermeture progressive contrôlée de l'incision lors du déclenchement du verrouillage dans au moins une position intermédiaire, et l'opération de verrouillage étant, si désiré, continuée au-delà de la position de départ de telle manière que les bords de l'incision avec la feuille de matériau fixée de manière adhésive et la peau sous-jacente soient inclinés vers le bas jusque dans la zone de l'incision et que les bords de la feuille de matériau de chaque côté de la ligne d'incision se touchent, et **par le fait que**, si désiré, après avoir éventuellement essuyé le côté supérieur du dispositif, une ou plusieurs feuilles de matériau supplémentaires (22) telles que revendiquées dans les revendications 35-40 soient fixées de manière adhésive afin de recouvrir la zone de l'incision, et de préférence également les moyens de fermeture (7, 8).
